# EUROPEAN PATENT APPLICATION

(11) **EP 4 193 846 A1**
(43) Date of publication of application: **14.06.2023**
(21) Application number: 21852913.9
(22) Date of filing: 01.06.2021
(51) Int. Cl.: A23L 3/26, A61L 2/08, A23L 15/00

(54) **STERILIZATION DEVICE AND STERILIZATION METHOD**

(30) Priority: 07.08.2020 JP 2020134382
(71) Applicant: Tokyo Metropolitan Industrial Technology Research Institute, Tokyo 135-0064 (JP)
(72) Inventor: KATAOKA, Noriaki, Tokyo 135-0064 (JP); KAWAHARA, Daigo, Tokyo 135-0064 (JP); SEKIGUCHI, Masayuki, Tokyo 135-0064 (JP)
(74) Representative: Morabito, Sara
(86) International application number: PCT/JP2021/020909
(87) International publication number: WO 2022/030087

(57) **Abstract**

A sterilizing device evenly irradiating an entire surface of a food with electron beam to provide a sterilizing effect based on the electron beam to the entire surface is provided. The sterilizing device includes: an electron-beam irradiator unit 10 configured to irradiate a process-target object (such as a raw egg) 40 with electron beam EB; a placement stage 22 for the process-target object 40; and a slit SL arranged between the process-target object 40 and the electron-beam irradiator unit 10, the slit unit SL includes a plurality of openings, and is arranged to be upper than a high portion of the process-target object 40. In this manner, since the process-target object 40 is irradiated with the electron beam EB through the slit SL, its entire surface can be more evenly irradiated with the electron beam, and a sterilizing effect based on the electron beam can be provided to the entire surface. The influence of the bremsstrahlung X ray caused by the irradiation with the electron beam on the inside contents of the process-target object 40 can be made small, and a dose of the X ray with which the inside contents (that is the portion to be the eatable part) of the process-target object 40 is irradiated can be made small so as to meet a criterion value regulated in a law or others.

## Description

### TECHNICAL FIELD

The present invention relates to a sterilizing device and a sterilizing method, and, more particularly relates to a technique effectively applied to a surface sterilizing process for a food having a shell or an outer skin covering an eatable part.

### BACKGROUND ART

In hen's egg, a cuticular layer on a surface of an eggshell is removed by washing, so that micropores (air holes) inside the eggshell are exposed. Because of this, microbes such as salmonella are easy to penetrate the micropores, and there is a risk of contamination of the washed egg surface.

Against such contamination, pharmaceutical sterilization (wet sterilization) is performed. However, in the pharmaceutical sterilization, there is concern about influence of residue of pharmaceuticals. For example, flavor such as smell can be deteriorated by the residue of pharmaceuticals. Also, there is concern about environmental pollution due to effluent.

Therefore, a process making use of ultraviolet ray, that is, a dry unheated process (dry sterilization) is practically applied. And, a process making use of radioactive ray, that is, a dry unheated process (dry sterilization) is studied.

For example, a Patent Document 1 discloses an electron-beam sterilizing device including: an electron-beam irradiator means with an irradiation window emitting electron beam; a delivery means delivering a parison (object) within an irradiation region of the electron beam emitted through the irradiation window; and magnets facing each other to interpose a delivery path therebetween for the parison delivered by the delivery means. According to the device, a magnetic field along an irradiation direction of the electron beam emitted through the irradiation window can be generated by the facing magnets, and the object can be efficiently sterilized by irradiating the parison with the electron beam emitted through the irradiation window and polarized by the magnetic field while the parison passes between the facing magnets to irradiate the parison.

A Patent Document 2 discloses a process method and a device for poultry eggs using an electron beam flux in sterilization of a calcareous shell. In the method including: a step of moving at least one egg through a beam path of an electronic beam source; and a step of irradiating the egg to irradiate the calcareous shell at various doses, the method further includes: a step of using an electron beam and widely irradiating all regions of the calcareous shell of the egg, and causing an element inserted into a path of the electron beam to distribute the irradiation over all regions of the calcareous shell to widely irradiate the calcareous shell at a dose being set within a predetermined target does range; a step of using an electron beam source and irradiating an egg that is rotating/already-rotated in the path of the electron beam; or a step of using an electron beam source and irradiating an egg that is held so that one side is positioned at zero degree inside a device arranged in an upstream of a rotating device.

### RELATED ART DOCUMENTS

### PATENT DOCUMENTS

Patent Document 1: Japanese Patent Application Laid-Open Publication No. 2017-209136
Patent Document 2: Japanese Patent Application Laid-Open Publication No. 2019-527046

### SUMMARY OF THE INVENTION

### PROBLEMS TO BE SOLVED BY THE INVENTION

As described above, methods of sterilizing foods are roughly classified into the wet sterilization and the dry sterilization. For example, in the case of the sterilization for only the surface of the hen's egg, wet sterilization making use of hypochlorous acid is applied. However, such a sterilizing method has problems of the residue of pharmaceuticals and the effluent as described above.

In the process making use of the ultraviolet ray (dry sterilization), the sterilizing effect is limited to only the surface of the hen's egg, and there is concern of failure to sufficiently sterilize an inside of the egg (such as an inside of the micropore in the eggshell). When dirt is adhered to the surface of the hen's egg, there is concern of failure in the sterilization.

On the other hand, in the process making use of the electron beam (dry sterilization), the sterilization is possible for not only the surface of the hen's egg but also the inside ranging from the eggshell to a certain depth. And, this method is effective since the electron beam can penetrate the dirt of the surface of the hen's egg and make the sterilization.

However, an end portion and a center portion of a surface of an oval sphere process target such as a hen's egg are different from each other in terms of a distance from an electron beam source, and therefore, cause a different degree of a surface dose at the time of the irradiation with the electron beam, and, as a result, it is difficult to evenly irradiate the entire surface of the hen's egg with the electron beam. And, increase in an irradiation intensity of the electron beam for providing the sterilizing effect to the entire surface of the hen's egg increases a dose of bremsstrahlung X ray generated by the irradiation with the electron beam, and undesirably increases an internal does of the hen's egg.

Meanwhile, for example, even a method of equalizing the surface dose of the electron beam by using the magnetic field as described above generates the bremsstrahlung X ray when the electron beam is bent, and therefore, undesirably increases the internal does of the hen's egg.

A purpose of the present invention is to provide a sterilizing device and a sterilizing method configured to evenly irradiate an entire surface of a food having an eggshell or an outer skin covering an eatable part with electron beam to provide a sterilizing effect based on the electron beam to the entire surface. Also, a purpose of the present invention is to provide a sterilizing device and a sterilizing method configured to suppress an X-ray irradiation dose on an eatable part of a food having an eggshell or an outer skin covering the eatable part to be low so as to satisfy a criterion value regulated in a law or others by reducing influence of bremsstrahlung X ray on the eatable part due to the irradiation with the electron beam while irradiating an entire surface of the food with electron beam.

### MEANS FOR SOLVING THE PROBLEMS

(1) A sterilizing device of the present invention is a sterilizing device configured to sterilize a process-target object by irradiating the process-target object with electron beam, the sterilizing device includes: an electron-beam irradiator unit configured to irradiate the process-target object with the electron beam; a placement unit for the process-target object; and a slit unit arranged between the process-target object and the electron-beam irradiator unit, and the slit unit includes a plurality of openings and is upper than a high portion of the process-target object.
(2) A sterilizing method of the present invention is a sterilizing method configured to sterilize a process-target object having an eatable part and a surface part covering the eatable part by irradiating the process-target object with electron beam, and the method includes: a step (a) of preparing an electron-beam irradiator including an electron-beam irradiator unit configured to irradiate the process-target object with the electron beam, a placement unit for the process-target object, and a slit unit arranged between the process-target object and the electron-beam irradiator unit; and a step (b) of placing the process-target object on the placement unit and irradiating the process-target object with the electron beam through the slit unit, and the slit unit of the step (b) includes a plurality of openings and is upper than a high portion of the process-target object.

### EFFECTS OF THE INVENTION

According to a sterilizing device or a sterilizing method of the present invention, an entire surface of a food having a shell or an outer skin covering an eatable part can be evenly irradiated with electron beam, and a sterilizing effect based on the electron beam can be provided to the entire surface. And, an X-ray irradiation dose on an eatable part of a food having an eggshell or an outer skin covering the eatable part can be suppressed to be low so as to satisfy the criterion value regulated in a law or others by reducing influence of bremsstrahlung X ray on the eatable part due to the irradiation with the electron beam while an entire surface of the food is irradiated with the electron beam.

### BRIEF DESCRIPTIONS OF THE DRAWINGS

FIG. 1 is a cross-sectional view and a perspective view showing an electron-beam irradiator and an electron-beam irradiating method of a first embodiment;
FIG. 2 is a cross-sectional view showing a state in which a process-target object is irradiated with electron beam not through a slit;
FIG. 3 is a schematic view showing a state of electron beam with which a slit or a metallic film is irradiated and a state of bremsstrahlung X ray;
FIG. 4 is a diagram showing a configuration of a raw egg;
FIG. 5 is a diagram showing a surface dose measuring model 1;
FIG. 6 is a diagram showing the surface dose measuring model 1;
FIG. 7 is a diagram showing a surface dose measuring model 2;
FIG. 8 is a diagram showing the surface dose measuring model 2;
FIG. 9 is a graph showing a surface dose of an irradiation specimen A;
FIG. 10 is a graph showing a surface dose based on simulation;
FIG. 11 is a graph showing an internal dose based on simulation;
FIG. 12 is a graph showing a surface dose;
FIG. 13 is a diagram showing a simulation result of the dose in vicinity of a surface of the egg in a case of usage of a slit 1 ("ϕ" of 1.5 mm, a pitch of 1.5 mm);
FIG. 14 is a graph showing a surface dose;
FIG. 15 is a diagram showing a simulation result of the dose in vicinity of the surface of the egg in a case of usage of the slit A;
FIG. 16 is a graph showing a measurement result of an internal dose of the internal dose measuring model 2 and a simulation result of the internal dose;
FIG. 17 is a cross-sectional schematic view showing an electron-beam irradiator of a second embodiment;
FIG. 18 is a perspective view showing a configuration of a delivery unit used in the electron-beam irradiator (sterilizing device) of the second embodiment;
FIG. 19 is a cross-sectional schematic view of the delivery unit in a Y direction;
FIG. 20 is a cross-sectional schematic view of the delivery unit in an X direction;
FIG. 21 is a diagram showing one example of a configuration of a support member;
FIG. 22 is a diagram showing one example of a configuration of a slit; and
FIG. 23 is a diagram showing relation between a height of an area of the process-target object and an area of an opening.

### BEST MODE FOR CARRYING OUT THE INVENTION

### (First Embodiment)

Hereinafter, embodiments of the present invention will be explained in detail on the basis of the drawings. In the following explanation, note that a term "A to B" means a term "equal to or larger than A and equal to or smaller than B" (the same goes for a second embodiment and others described later).

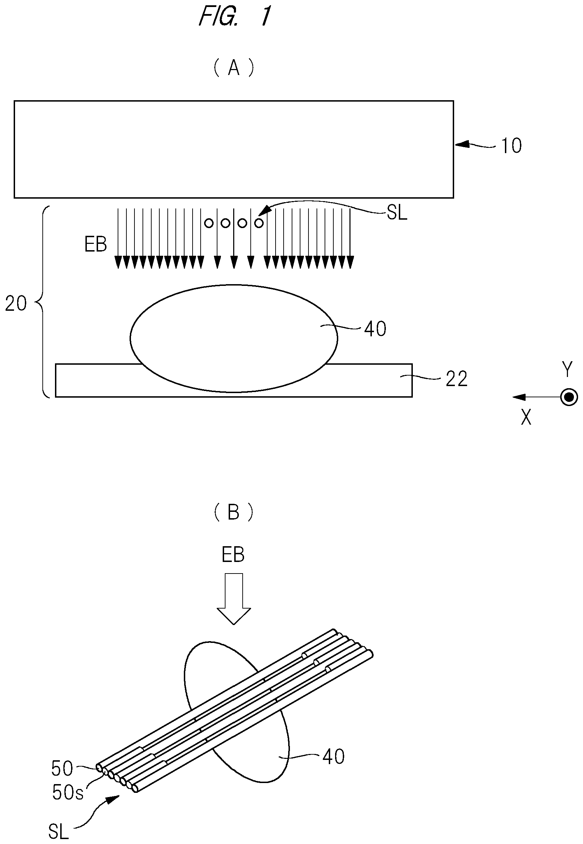

FIG. 1 is a cross-sectional view and a perspective view showing an electron-beam irradiator and an electron-beam irradiating method of the present embodiment.

The electron-beam irradiator (sterilizing device) shown in FIG. 1(A) includes an electron-beam generator unit 10 and an irradiation chamber 20. A process-target object (irradiation-target object or process target such as a raw egg) 40 placed on a placement stage (placement unit) 22 of an irradiation chamber 20 is irradiated with electron beam EB generated from the electron-beam generator unit 10. Regarding a detailed configuration of the electron-beam irradiator (sterilizing device), see explanation for a second embodiment.

In the present embodiment, when a process-target object 40 is irradiated with the electron beam EB through the slit (slit unit, slit member) SL, its entire surface can be evenly irradiated with the electron beam, so that the sterilizing effect based on the electron beam can be provided to the entire surface. And, the influence of the bremsstrahlung X ray on the inside contents (eatable part) of the process-target object caused by the irradiation with the electron beam can be made small.

As shown in FIG. 1(B), the slit SL includes a metallic bar 50 and a metallic spacer 50s. For example, four long circular bars and six short circular bars each of which is an aluminium circular bar having a diameter of 1.5 mm (also referred to as ϕ of 1.5 mm in some cases) are prepared, and the short circular bar is arranged as the spacer on both ends of the long circular bar between the long circular bars. In this manner, the slit SL having ϕ of 1.5 mm and the pitch of 1.5 mm can be formed.

In this case, the metallic bar 50 and the metallic spacer 50s are made of the same metal. However, these members may be made of a metal different from each other. When the diameters ϕ of the metallic bar 50 and the metallic spacer 50s are changed, combination of a width of the metallic bar 50 that is a shielding portion and a width of the metallic spacer 50s that is an opening can be adjusted. In place of the metallic members, a carbon bar 50 and a carbon spacer 50s may be used. Alternatively, the slit SL may be made of combination of the metallic bar and the carbon spacer or combination of the carbon bar and the metallic spacer.

Alternatively, as the slit SL, a quadrangular metallic panel with a plurality of linear openings may be used. For example, when the linear openings each having a width of 1.5 mm are arranged at a pitch of 1.5 mm, the slit SL having the same shape as that of FIG. 1 (B) can be provided.

As a constituent material of the slit SL, it is preferable to use a material having a favorable electric conductivity such as metal (such as aluminium (Al), iron (Fe), copper (Cu), lead (Pb), titanium (Ti) and tungsten (W)), carbon (C) or others. And, it is preferable to use a material having a small thermal expansion coefficient.

This slit SL is arranged on the highest portion (also referred to as high portion) of the process-target object (in this case, the raw egg) 40. In the case of the raw egg, the high portion is a substantially center portion in a plan view viewed from above.

As described above, according to the present embodiment, since the process-target object 40 is irradiated with the electron beam EB through the slit SL, its entire surface can be evenly irradiated with the electron beam, and the sterilizing effect based on the electron beam can be provided to its entire surface. And, an X-ray irradiation dose on an inside (that is a part to be an eatable part) of the process-target object 40 can be suppressed to be low so as to satisfy the criterion value regulated in a law or others by reducing influence of bremsstrahlung X ray on the process-target object 40 due to the irradiation with the electron beam while an entire surface of the process-target object is irradiated with the electron beam.

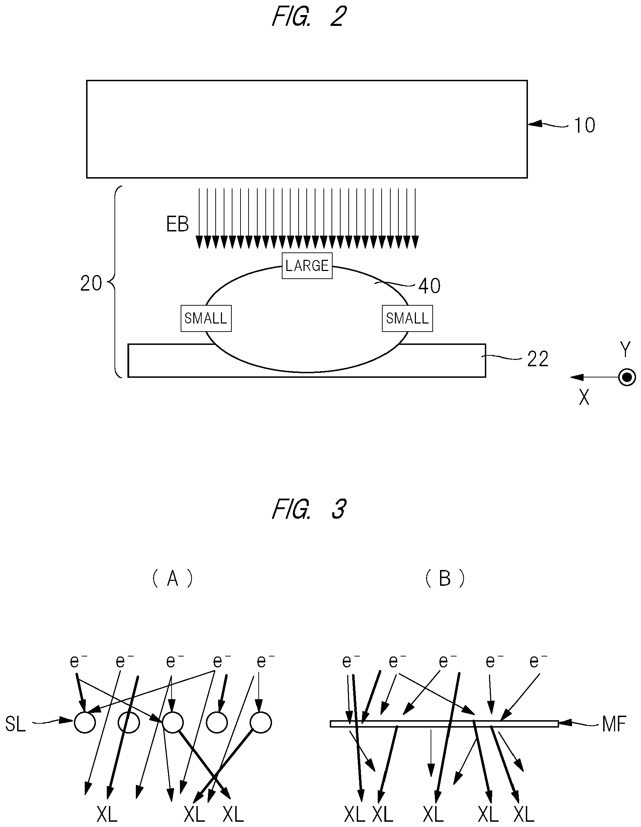

On the other hand, when the process-target object 40 is directly irradiated with the electron beam not through the slit SL as shown in FIG. 2, the intensity of the electron beam is large at the high portion of the process-target object (in this case, the raw egg) 40 while the intensity of the electron beam is small at a low portion of the process-target object (in this case, the raw egg) 40. Therefore, if the necessary intensity of the electron beam for the sterilization is set to be suitable for the portion (high portion) at which the intensity of the electron beam is large, the sterilization performance decreases at the low portion. On the other hand, if the necessary intensity of the electron beam for the sterilization is set to be suitable for the portion (low portion) at which the intensity of the electron beam is small, the intensity of the electron beam at the high portion is too large, and the internal dose due to the bremsstrahlung X ray is particularly large. FIG. 2 is a cross-sectional view showing a state in which the process-target object is irradiated with the electron beam not through the slit. Note that the high portion of the process-target object (in this case, the raw egg) 40 corresponds to a portion (center portion) having a small distance between the process-target object and the electron-beam irradiator (electron-beam irradiator unit) while the low portion of the process-target object (in this case, the raw egg) 40 corresponds to a portion (end portion) having a large distance between the process-target object and the electron-beam irradiator (electron-beam irradiator unit). Since the slit SL is arranged in almost parallel to the electron-beam irradiator unit, the height of the process-target object (in this case, the raw egg) 40 may be set based on the distance between the process-target object and the slit SL.

FIG. 3 is a schematic view showing a state of the electron beam with which the slit or the metallic film is irradiated and a state of the bremsstrahlung X ray. In this case, a method of irradiation with the electron beam EB through a metallic film MF is exemplified as a method for decreasing the dose on the high portion of the process-target object (in this case, the raw egg) 40. However, in the case of the usage of the metallic film MF, the irradiation electrons collide with metallic atoms configuring the metallic film MF, and are scattered. Therefore, energy of the electrons decreases, and the dose on the surface of the process-target object undesirably decreases. Further, an occurrence ratio of the bremsstrahlung X ray is increased by the scattering of the electrons (FIG. 3(B)).

On the other hand, in the case of the usage of the slit SL as shown in FIG. 3(A), a metallic portion and a spatial portion exist, and the process-target object is irradiated while the electrons passing the spatial portion hold the energy. Also, when a certain or larger distance between the slit SL and the process-target object is maintained, evenness of a surface dose of the electrons passing the spatial part is achieved. Further, since the occurrence ratio of the bremsstrahlung X ray is lower than that of the metallic film MF, the usage of the slit SL is more effective.

As described above, in the present embodiment, the object is irradiated with the electron beam through the slit SL, and, as a result, its entire surface can be more evenly irradiated with the electron beam, and the sterilizing effect based on the electron beam can be provided to its entire surface. Also, the surface of the process-target object can be efficiently sterilized without the increase of the internal dose (particularly the absorbed dose of the X ray (bremsstrahlung X ray) XL).

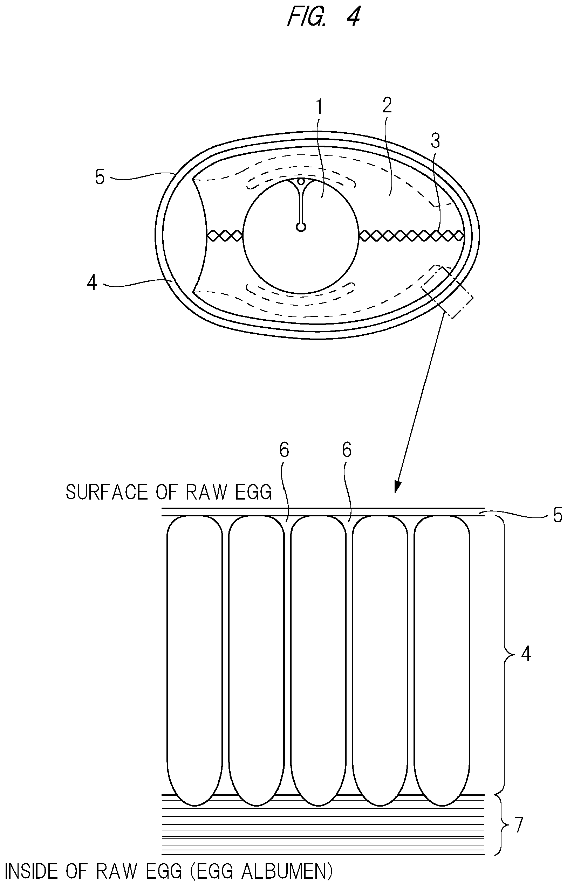

Specifically, when an eggshell of the raw egg (process-target object 40) having a yolk 1 and an albumen 2 is irradiated with the electron beam EB through the slit SL from the electron-beam irradiator unit 10, a part having a certain depth from the surface of the eggshell can be sterilized (such as the sterilization of salmonella) (see FIG. 4). In the irradiation of the electron beam through the slit, irradiation time for achieving the predetermined surface dose increases, and therefore, the absorbed dose of the X ray (bremsstrahlung X ray) XL on the eatable part (the yolk 1 and the albumen 2) inside the eggshell increases. However, because of the effect of the slit shielding the X ray, the dose can be decreased to satisfy, for example, the criterion value (such as 0.1 Gy = 100 mGy) regulated in a law or others without the extreme increase of the absorbed dose of the X ray. Note that the irradiation with the electron beam of 1 kGy decreases the amount of the salmonella down to 1/10000, and the irradiation with the electron beam of 3 kGy decreases the amount of the salmonella down to below the detection limit.

### [Working Examples]

The sterilizing process for the raw egg, making use of the electron-beam irradiator (sterilizing device) and the sterilizing method of the present embodiment, will be more specifically explained below with reference to working example.

First, a shape of the raw egg will be explained.

FIG. 4 is a diagram showing the configuration of the raw egg. As shown in FIG. 4, the raw egg has the eggshell 4, and the yolk 1 and the albumen 2 inside the eggshell. The yolk 1 and the albumen 2 are connected to each other by a chalaza 3. There is a cuticular layer 5 outside the eggshell 4, and there is an eggshell membrane 7 on an inner wall of the eggshell 4. The eggshell 4 has a plurality of pores 6. The cuticular layer 5 can be removed by being washed. A blunt end of the raw egg has an air cell. The following simulation specimen was prepared in consideration of such a raw-egg structure.

A low-energy electron accelerator (EC-250: produced by IWASAKI ELECTRIC CO., LTD.) was used as the electron-beam irradiator, and the irradiation with the electron beam having an acceleration voltage of 120 kV was performed. The irradiation with the electron beam was performed while the slit SL was arranged below an irradiation unit (irradiation window) of the low-energy electron accelerator.

### <Surface Dose Measuring Model 1>

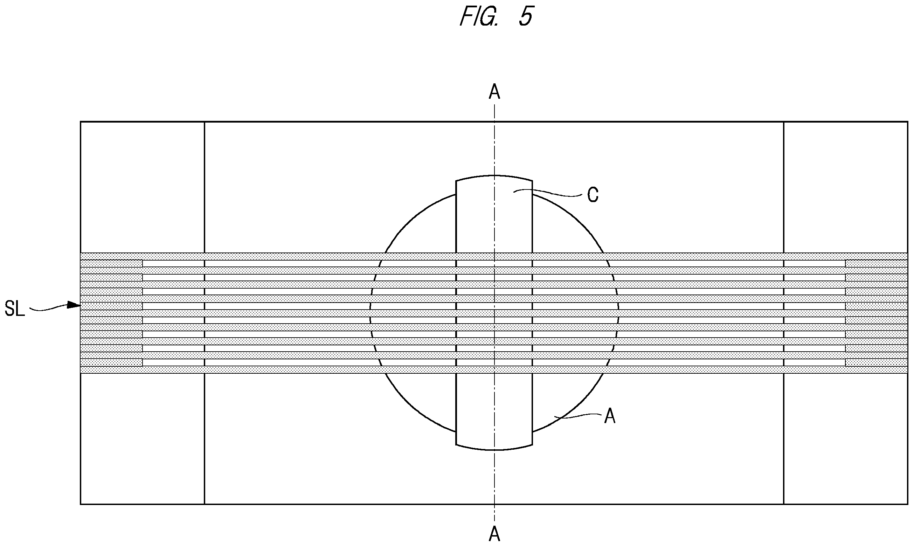

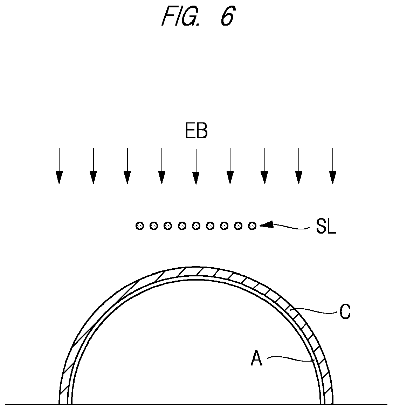

A model 1 using the following irradiation specimen and slit was prepared in order to evaluate the surface absorbed dose. Each of FIGs. 5 and 6 is a diagram showing the surface dose measuring model 1. FIG. 5 is a top view, and FIG. 6 is a cross-sectional view corresponding to a portion A-A of FIG. 5.

The raw egg, a surface of which was washed, (washed egg, M size) was prepared. A blunt-end eggshell was obtained by cracking of the raw egg to be along an outer circumference in a minor radius direction, followed by removal of the inner contents and the eggshell membrane and drying of the egg. A chemical process indicator label (Crosstex) C was wound around and pasted on a center portion of the outer circumference of the blunt-end eggshell to prepare the eggshell simulation specimen (raw-egg simulation sample, irradiation specimen) A.

Nine long circular bars and sixteen short circular bars each of which is an aluminium circular bar having ϕ of 1.5 mm were prepared, and the slit SL having ϕ of 1.5 mm and the pitch of 1.5 mm was formed.

The slit SL was arranged above the high portion of the eggshell simulation specimen A to cross the chemical process indicator label C.

A low-energy electron accelerator (EC-250: produced by IWASAKI ELECTRIC CO., LTD.) was used, and the irradiation with the electron beam EB having the acceleration voltage of 120 kV was performed. The distance between the slit SL and the eggshell simulation specimen A was set to 3 mm.

### <Internal Dose Measuring Model 2>

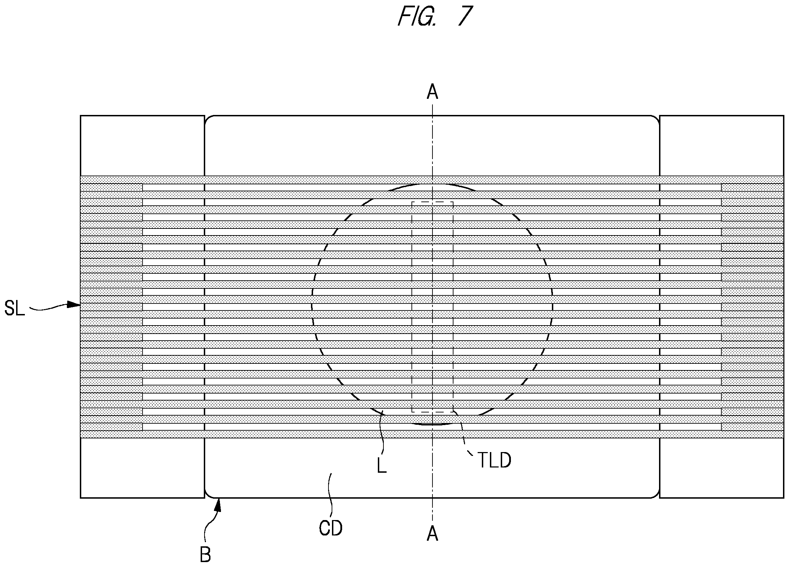

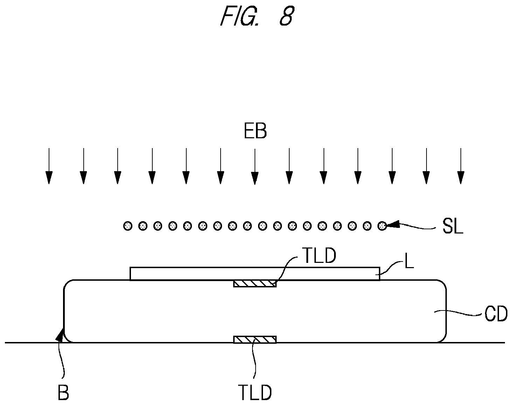

A model 2 using the following irradiation specimen and slit was prepared in order to evaluate the internal absorbed dose (the dose of the bremsstrahlung X ray). Each of FIGs. 7 and 8 is a diagram showing the internal dose measuring model 2. FIG. 7 is a top view, and FIG. 8 is a cross-sectional view corresponding to a portion A-A of FIG. 7.

A liquid agar of 2% was poured in a Tupperware (food storage container) and was solidified, and then, was cut to have a size of "50 mm × 70 mm" to prepare an agar "CD" having a thickness of 13 mm. Five TLD elements (TLD100: produced by Thermo Fisher Scientific, Inc.) each upper portion and lower portion of which are shielded with a polyethylene film were arranged and were covered with a plastic wrap as a whole. A circular calcium carbonate layer L having a thickness of 0.4 mm and a diameter of 2.0 cm was formed in the upper portion of the agar to prepare the egg simulation specimen (raw-egg simulation sample, irradiation specimen) B. The calcium carbonate layer L corresponds to the eggshell, and the agar CD corresponds to the inside contents (eatable part, yolk and albumen) of the raw egg.

Eighteen long circular bars and thirty four short circular bars (spacers) each of which is an aluminium circular bar having ϕ of 1.5 mm were prepared, and a slit SLa having ϕ of 1.5 mm and the pitch of 1.5 mm was formed. Also, twenty five long circular bars and forty eight short circular bars each of which is an aluminium circular bar having ϕ of 1.0 mm were prepared, and a slit SLb having ϕ of 1.0 mm and the pitch of 1.0 mm was formed.

The slit SL was arranged above the egg simulation specimen B to cross the arrangement direction of the TLD element.

The low-energy electron accelerator (EC-250: produced by IWASAKI ELECTRIC CO., LTD.) was used, and the irradiation with the electron beam EB having the acceleration voltage of 120 kV was performed. The distance between the slit SL and the eggshell simulation specimen B was set to 3 mm.

### <Simulation>

The Particle and Heavy Ion Transport code System PHITS (Version 2.96) was used for creation of the model in conformity with the configurations of the models 1 and 2. A density of the eggshell (main component: CaCO₃) was set to 2.0 because of having pores, a density of the eatable part was set to be equal to that of water, atmosphere was set to the standard air, and the dose distributions of the electron beam and the bremsstrahlung X ray in the irradiation with the electron beam on the egg were evaluated under the Monte Carlo simulation. As parameters, the acceleration voltage, a slit position (length), the distance between the slit and the process-target object, the diameter of the circular bar configuring the slit, the pitch of the circular bar, the material of the circular bar and others are conceivable. From the simulation under selection of some of these parameters, relation between each parameter and the dose (surface dose, internal dose) was revealed.

### [Working Example 1]

The surface dose of the irradiation specimen A was measured in the surface dose measuring model 1 (FIGs. 5 and 6).

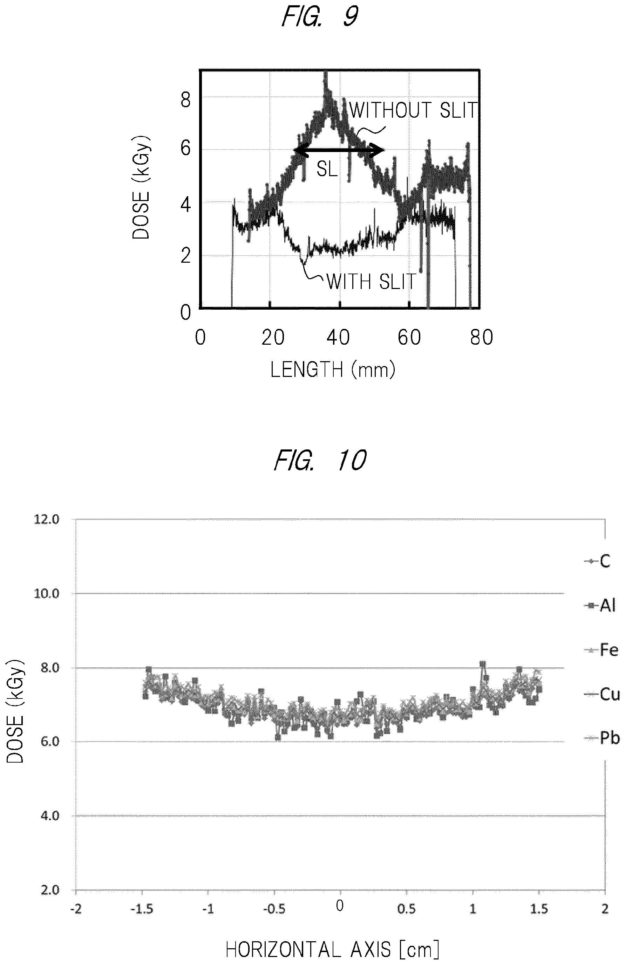

FIG. 9 is a graph showing the surface dose of the irradiation specimen A. A horizontal axis indicates the length (position, mm), and a vertical axis indicates the dose (kGy). An arrow indicates the arrangement position of the slit SL. As shown in FIG. 9, in the case "with the slit" in comparison to the case "without the slit", it was verified that the surface dose decreases in vicinity of the high portion of the irradiation specimen A, and therefore, it was verified that the evenness of the surface dose is increased by the slit SL.

### [Working Example 2]

In the working example 1, aluminium was used as the constituent material of the slit. However, cases of usages of carbon (C), aluminium (Al), iron (Fe), copper (Cu) and lead (Pb) as the constituent material of the slit were evaluated using the simulation. The surface dose was calculated using the parameters in conformity with the model 1 other than the material of the circular bar.

FIG. 10 is a graph showing the surface dose based on the simulation. A horizontal axis indicates the length (position, cm), and a vertical axis indicates the dose (kGy). As shown in FIG. 10, it was verified that the evenness of the surface dose can be achieved in all cases of the usages of carbon (C), aluminium (Al), iron (Fe), copper (Cu) and lead (Pb) as the constituent material of the slit. Among carbon (C), aluminium (Al), iron (Fe), copper (Cu) and lead (Pb) as the material of the slit, there is no difference in the effect of the evenness of the surface dose.

### [Working Example 3]

In the working example 1, aluminium was used as the constituent material of the slit. However, the internal dose in each of cases of usages of carbon (C), aluminium (Al), titanium (Ti), iron (Fe), and tungsten (W) as the constituent material of the slit was evaluated based on the simulation. The internal dose was calculated using the parameters in conformity with the model 2 other than the material of the circular bar.

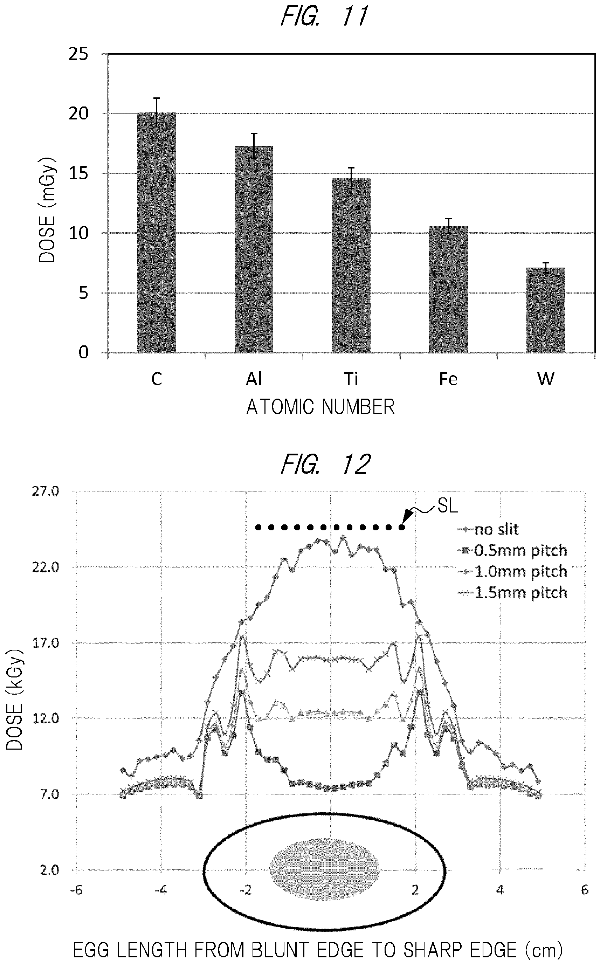

FIG. 11 is a graph showing the internal dose based on the simulation. A horizontal axis indicates a chemical element, and a vertical axis indicates the dose (mGy). The chemical elements are aligned in an ascending order of an atomic number from left. As shown in FIG. 11, in the cases of the usages of carbon (C), aluminium (Al), titanium (Ti), iron (Fe) and tungsten (W) as the constituent material of the slit, the larger the atomic number of the constituent material of the slit is, the smaller the internal dose is. Even in the low-energy electron beam irradiation on the egg, it was revealed that the X-ray amount on the eatable part can be decreased by the usage of the slit made of a material (particularly a metal) having a large atomic number.

### [Working Example 4]

Influence of the slit pitch in the irradiation with the electron beam through the slit was evaluated based on the simulation. Specifically, a plurality of aluminium circular bars having ϕ of 1.5 mm were used as the slit, and the surface dose was measured using a slit 1 (ϕ of 1.5 mm, pitch of 1.5 mm) arranged at a pitch of 1.5 mm, a slit 2 (ϕ of 1.5 mm, pitch of 1.0 mm) arranged at a pitch of 1.0 mm and a slit 3 (ϕ of 1.5 mm, pitch of 0.5 mm) arranged at a pitch of 0.5 mm. The surface dose was calculated using the parameters in conformity with the model 1 other than the pitch of the circular bar. The surface dose in the case without the slit (no slit) was also similarly measured.

FIG. 12 is a graph showing the surface dose. A horizontal axis indicates the length (position, cm), and a vertical axis indicates the dose (Dose, kGy). The slit is arranged between a position of -2 cm and a position of 2 cm. As shown in FIG. 12, in the case without the slit (no slit), the smaller from 1.5 mm to 0.5 mm the pitch is, the smaller the value of the surface dose is. From this, it was revealed that the smaller the slit pitch is, the larger the effect of the suppression of the surface dose is.

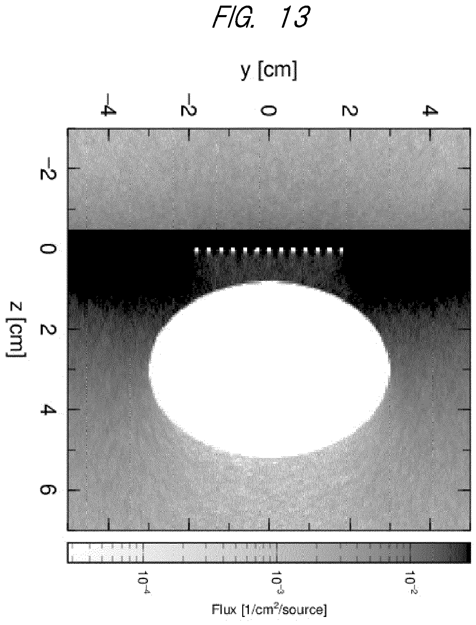

FIG. 13 is a diagram showing simulation results of the dose in vicinity of the egg surface in the case of the usage of the slit 1 (ϕ of 1.5 mm, pitch of 1.5 mm). As illustrated in the drawing, the color is paler in a lower portion of the slit 1 (SL) than an upper portion of the slit 1, and it is revealed that the dose is suppressed by the slit 1 (SL).

In this case, in the graph of FIG. 12, a shape of the graph in each of the case of the slit 1 (ϕ of 1.5 mm, pitch of 1.5 mm) and the case without the slit (no slit) is similar to the shape of the graph shown in FIG. 9, and therefore, it is revealed that the simulation result used in the present working example extremely matches with the result of the working example based on the specific experiment.

### [Working Example 5]

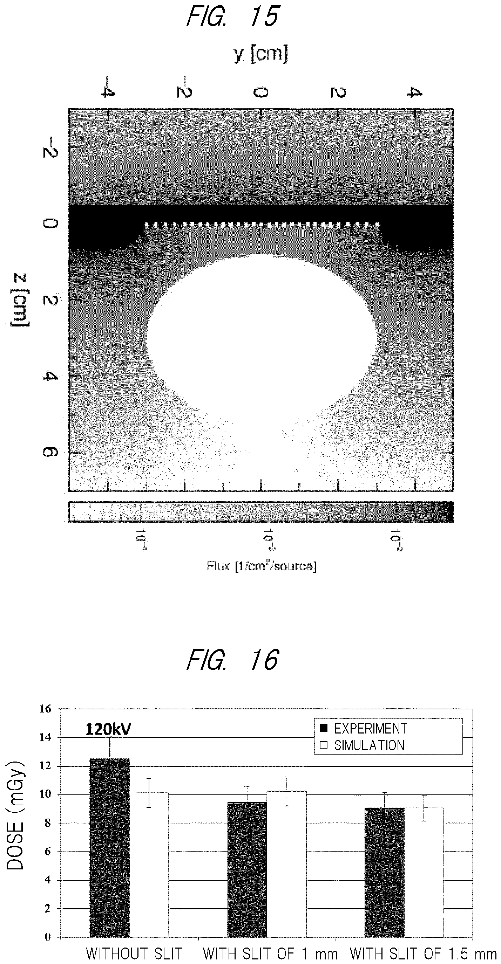

Influence of the change of the slit pitch in the irradiation with the electron beam through the slit was evaluated based on the simulation. Specifically, a plurality of tungsten circular bars having ϕ of 1.5 mm were arranged as the slit (see FIG. 15), and the surface dose was measured using a slit A changing in the pitch on its center portion and both ends and a slit B (ϕ od 1.5 mm, pitch of 1.5 mm) having a constant pitch. The slit A having the pitch of the center portion of 0.5 mm and the pitch of the both ends of 1.0 mm was used. The surface dose was calculated using the parameters in conformity with the model 1 other than the pitch of the circular bar.

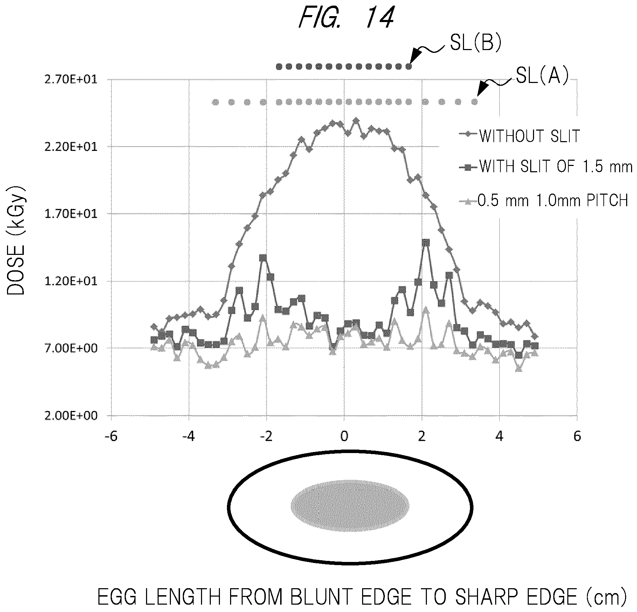

FIG. 14 is a graph showing the surface dose. A horizontal axis indicates the length (position, mm), and a vertical axis indicates the dose (Dose, kGy). The slit is arranged between a position of -2 cm and a position of 2 cm or a position of -4 cm and a position of 4 cm. As shown in FIG. 14, the value of the surface dose is smaller in the case of the usage of the slit B (ϕ of 1.5 mm, pitch of 1.5 mm) than the case without the slit (no slit), and besides, the value of the surface dose is much smaller in the case of the usage of the slit A. The further evenness of the value of the surface dose is achieved in the case of the usage of the slit A than the case of the usage of the slit B.

As seen from this, it is revealed that the further evenness of the surface dose is achieved when the slit pitch at the center portion (high portion) of the egg is densified while the slit pitch at the end portion (low portion) of the egg is isolated (made less dense).

FIG. 15 is a diagram showing simulation results of the dose in vicinity of the egg surface in the case of the usage of the slit A. As illustrated in the drawing, the color is paler in a lower portion of the slit A (SL) than an upper portion of the slit A (SL), and it is revealed that the dose is suppressed by the slit A. The color is stronger in a portion immediately below the both ends of the slit A (SL) than a portion immediately below the center portion of the slit A (SL), and therefore, it is revealed that the further evenness of the color strength of the egg surface is achieved.

### [Working Example 6]

The internal dose of the irradiation specimen B was measured based on the internal dose measuring model 2. The internal dose in the case without the slit was also similarly measured. Also, the internal dose was calculated based on the simulation using the parameters in conformity with the model 2. The internal dose in the case without the slit was also similarly measured based on the simulation.

FIG. 16 is a graph showing the measuring result of the internal does of the internal dose measuring model 2 and the simulation result of the internal does. The internal does of the TLD element (see FIGs. 7 and 8) above the agar in the case of the usage of the slit SL having ϕ of 1.5 mm and the pitch of 1.5 mm was about 9 mGy, the internal does of the TLD element above the agar in the case of the usage of the slit SL having ϕ of 1.0 mm and the pitch of 1.0 mm was about 9.5 mGy, and the internal does of the TLD element above the agar in the case without the slit was about 12.5 mGy. The matching between the resultant actual measurement values in the present working example and the results based on the simulation was high.

In the working examples 1 to 6, the specific measurement results of both the surface dose and the internal dose match with the simulation results, and therefore, it is revealed that the slit can be designed based on the simulation.

### (Summary of Process Condition)

In the working examples, the acceleration voltage was set to 120 kV. However, the acceleration voltage can be adjusted in a range that is, for example, equal to or higher than 80 kV and equal to or lower than 150 kV.

The surface dose can be adjusted in a range that is equal to or higher than 0.1 kGy and equal to or lower than 10 kGy.

The internal dose is preferable to be equal to or lower than 0.1 kGy (= 100 mGy) that is an allowable criterion value of the internal dose in the test for food or others. Note that the measurement results of the internal dose explained in the working example 6 are significantly lower than this criterion value.

### (Second Embodiment)

In the present embodiment, an example of irradiation with the electron beam through the slit at the same time of rotation of the process-target object will be explained.

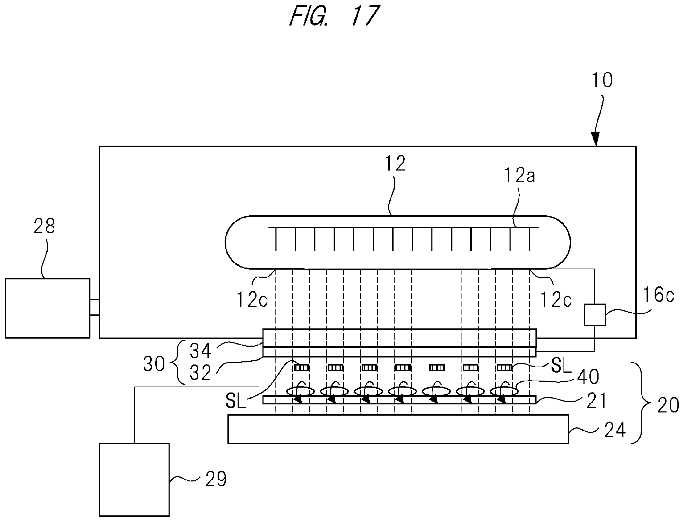

FIG. 17 is a schematic cross-sectional view showing an electron-beam irradiator of the present embodiment.

The electron-beam irradiator (sterilizing device) shown in FIG. 17 includes an electron-beam generator unit 10 and an irradiation chamber 20. The process-target object (irradiation-target object such as a raw egg) 40 arranged in the irradiation chamber 20 is irradiated with the electron beam through the irradiation window unit (irradiation window) 30 arranged in the electron-beam generator unit 10. The process-target object 40 is arranged on a delivery unit 21 such as a conveyor and is delivered. In the present embodiment, the process-target object 40 is delivered while rotating on the delivery unit 21. When the process-target object 40 that is delivered while rotating is irradiated with the electron beam through the slit SL, its entire surface is evenly irradiated with the electron beam, so that the sterilizing effect based on the electron beam can be provided to the entire surface. As a result, the influence of the bremsstrahlung X ray caused by the irradiation with the electron beam on the inside contents (eatable part) of the process-target object can be made small.

Note that the structure of the slit SL can be configured to be the same structure as the first embodiment, and therefore, a structure other than that of the slit SL will be explained in detail herein.

The electron-beam generator unit 10 includes a terminal 12 generating the electron beam inside a chamber and a space (acceleration space) where the electron beam that is generated in the terminal 12 is accelerated. And, the space (acceleration space) of the electron-beam generator unit 10 is kept in a vacuum state of about 10⁻³ to 10⁻⁵ Pa by a vacuum exhaust system 28 in order to prevent energy loss due to collision of the electrons with gas molecules and prevent oxidation of a filament 12a. The terminal 12 includes the linear filament 12a releasing thermal electrons and a grid 12c controlling the thermal electrons generated in the filament 12a.

And, the electron-beam generator unit 10 includes a heating power supply (not illustrated) for use in heating the filament 12a to generate the thermal electrons, a controlling direct-current power supply (not illustrated) applying a voltage to a gap between the filament 12a and the grid 12c, and an accelerating direct-current power supply 16c applying a voltage (acceleration voltage) to a gap between the grid 12c and a window foil 32 arranged in the irradiation window unit 30.

The irradiation chamber 20 includes an irradiation space where the process-target object (irradiation-target object) 40 is irradiated with the electron beam. The process-target object 40 is delivered through the irradiation chamber 20 by a delivery unit 21 such as a conveyor in a direction, for example, from a depth side of a sheet of FIG. 1 toward a front side of the same. A beam collector 24 is arranged inside the irradiation chamber 20. This beam collector 24 absorbs the electron beam penetrating the process-target object 40. Note that the electron-beam generator unit 10 and the irradiation chamber 20 are covered by a lead shielding plate so as to prevent the X ray that is secondarily generated at the time of the irradiation with the electron beam from leaking out.

The inside of the irradiation chamber 20 is under atmosphere of an inert gas, ambient air or others in accordance with a process content. In the case of the sterilizing process (disinfection process), the irradiation atmosphere inside the irradiation chamber 20 is set to be under the ambient air (atmosphere containing oxygen), and the process-target object is sterilized by the electron beam. In this case, ozone is generated from the oxygen by the electron-beam irradiation, and then, is reacted with nitrogen in the ambient air, so that NOx is generated. Since the NOx corrodes the window foil 32, the generation of the NOx can be controlled by blowing of dry air from a blower 29 into the irradiation chamber 20.

The irradiation window unit (irradiation window) 30 includes the window foil 32 and a window frame unit 34 made of copper. The window frame unit 34 is used for supporting the window foil 32. In the window frame unit 34, a rectangularshaped (quadrangular-shaped) opening is formed. A width of the opening is, for example, about 1 cm, and its longitudinal direction is arranged in a direction that is orthogonal to the deliver direction.

And, inside the irradiation window unit 30, a cooling passage (not illustrated) is formed in order to cool the window foil 32, a temperature of which is increased by the electron-beam irradiation. The window frame unit 34 is detachable to the irradiation opening of the electron-beam generator unit 10. The window foil 32 is detachably adhered to a lower surface of the window frame unit 34. As the window foil 32, a metal foil such as an aluminum foil or a titanium (Ti) foil is used.

In the electron-beam irradiator of the present embodiment, when the filament 12a is heated through the electric current by the heating power supply, the filament 12a releases the thermal electrons, and the released thermal electrons are attracted everywhere by a control voltage of the controlling direct-current power supply applied between the filament 12a and the grid 12c. Among the thermal electrons, only thermal electrons that pass the grid 12c are effectively extracted as the electron beam. Then, the electron beam that is extracted from the grid 12c is accelerated in the acceleration space by an acceleration voltage of the accelerating direct-current power supply 16c applied between the grid 12c and the window foil 32, and then, penetrates the window foil 32, and the process-target object that is delivered through the irradiation chamber 20 below the irradiation window unit 30 while rotating is irradiated with the electron beam. Note that an electric current value generated by the flow of the electron beam extracted from the grid 12c is referred to as beam electric current. The larger the beam electric current is, the more the electron beam is.

In the electron-beam irradiator, predetermined values are set to the acceleration voltage, the beam electric current, a delivery speed (irradiation time) of the process-target object, a distance between the electron-beam irradiator unit and the process-target object and others, and then, the electron-beam irradiation process is performed to the process-target object. The energy applied on the electron beam is defined by the acceleration voltage. In other words, the higher the set acceleration voltage is, the larger the resultant kinetic energy of the electron beam is. As a result, the electron beam can reach a position deep from the surface of the process-target object. Therefore, by the change in the setting value of the acceleration voltage, the penetrating depth of the electron beam in the process-target object can be adjusted.

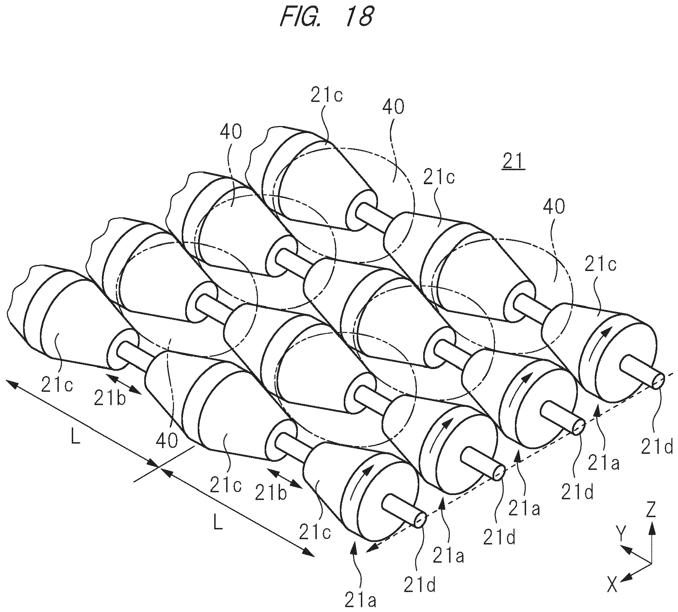

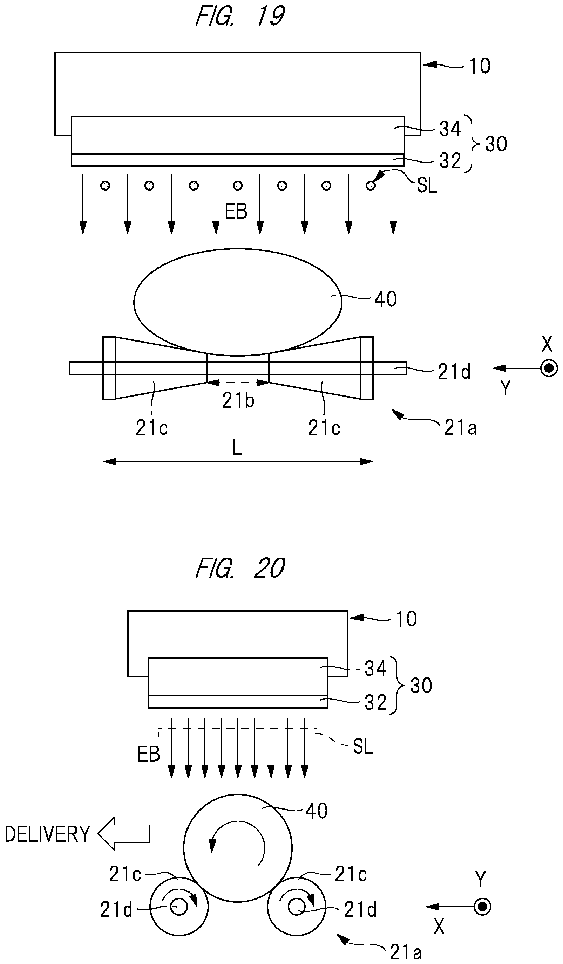

FIG. 18 is a perspective view showing a configuration of the delivery unit for use in the electron-beam irradiator (sterilizing device) of the present embodiment, and FIGs. 19 and 20 are cross-sectional schematic views of the delivery unit in a Y direction and an X direction, respectively.

As shown in FIGs. 18 to 20, the delivery unit 21 has a plurality of rollers 21a extending in the Y direction that is orthogonal to the delivery direction (in this case, the X direction). The roller 21a has a plurality of lanes L. The roller 21a has a shaft 21d and circular truncated cone members 21c arranged on both ends of the shaft 21d at the lane L, and there is a space (gap) 21b between the circular truncated cone members 21c. The circular truncated cone member 21c is arranged so as to be gradually thinner toward the space (gap) 21b.

The process-target object (in this case, the raw egg) 40 is placed at each lane L so as to straddle these circular truncated cone members 21c on both sides of the space (gap) 21b. When the circular truncated cone member 21c rotates about the shaft 21d, the process-target object (in this case, the raw egg) 40 rotates. The shaft 21d of the roller is joined to a chain or a belt (not illustrated) so as to be rotatable. By movement of this chain in the delivery direction (in this case, the X direction, see a dashed arrow), the process-target object (in this case, the raw egg) 40 is delivered in the X direction while rotating.

Therefore, when the electron-beam irradiator as shown in FIG. 17 is used, the sterilizing process can be performed to the surface of the food by the electron-beam irradiation through the slit SL while the food having the shell or the outer skin covering the eatable part that is the process-target object 40 is rotated. The absorbed dose of the X ray (bremsstrahlung X ray) XL on the eatable part (the yolk 1 and the albumen 2) of the food can be small so as to, for example, meet the criterion value (such as 0.1 Gy = 100 mGy) regulated in a law or others without locally increasing.

### (Summary of Process Conditions)

Various process conditions of the present embodiment will be explained.

Also in the present working example, the acceleration voltage can be adjusted to be lower than 1 MV such as a range that is, for example, equal to or higher than 80 kV and equal to or lower than 150 kV.

The surface dose can be adjusted in a range that is, for example, equal to or higher than 0.1 kGy and equal to or lower than 10 kGy.

The internal dose is preferably equal to or lower than 0.1 Gy (= 100 mGy) that is the allowable internal dose criterion value in the test for the food or others.

In consideration of the impact on the food, the rotation speed of the food that is the process-target object is preferably 0.3 rotation/seconds to 5 rotation/seconds.

In consideration of the sterilizing speed, the delivery speed of the food that is the process-target object is preferably 0.1 m/minutes to 5 m/minutes.

### (Third Embodiment)

In the present embodiment, various application examples according to the above-described embodiments will be explained.

### (First Application Example)

In the first embodiment, the aluminium circular bar was used as the slit SL. However, as described above, as the constituent member of the slit, a metal other than aluminium may be used, or an alloy, metallic compound or others may be used. Other than the metal, carbon may be used, or a multilayered structure made of metal or carbon may be used. It is particularly preferable to use a material having a small thermal expansion and a large thermal conductivity.

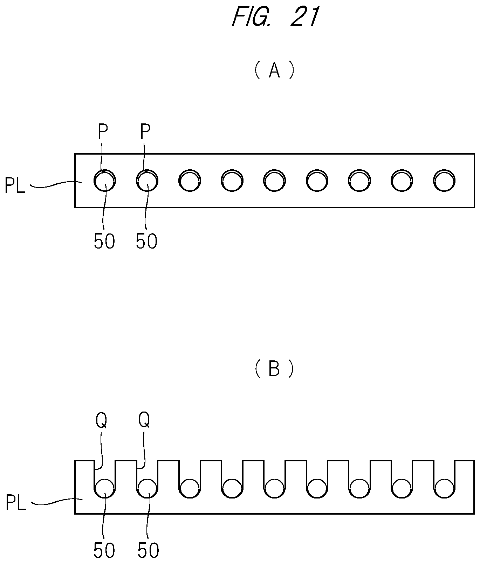

The cross-sectional shape of the bar may be not the circular shape but ellipsoidal, quadrangular (square, rectangular) or other polygonal shape. In place of the spacer, a circular bar supporting member may be used. FIG. 21 is a diagram showing one example of a configuration of the supporting member. For example, as shown in FIG. 21(A), a supporting member PL including a hole P in which a circular bar 50 is inserted may be used. As shown in FIG. 21(B), a supporting member PL including a concave portion Q on which the circular bar 50 is placed may be used. A size of the hole P or the concave portion Q is made larger than a cross section of the circular bar 50, and, as a result, deformation or distortion of the slit can be prevented even if the circular bar 50 is thermally expanded.

### (Second Application Example)

In the first embodiment, the aluminium circular bar was used as the slit SL. However, as described above, a quadrangular metallic plate having a plurality of linear openings OA may be used.

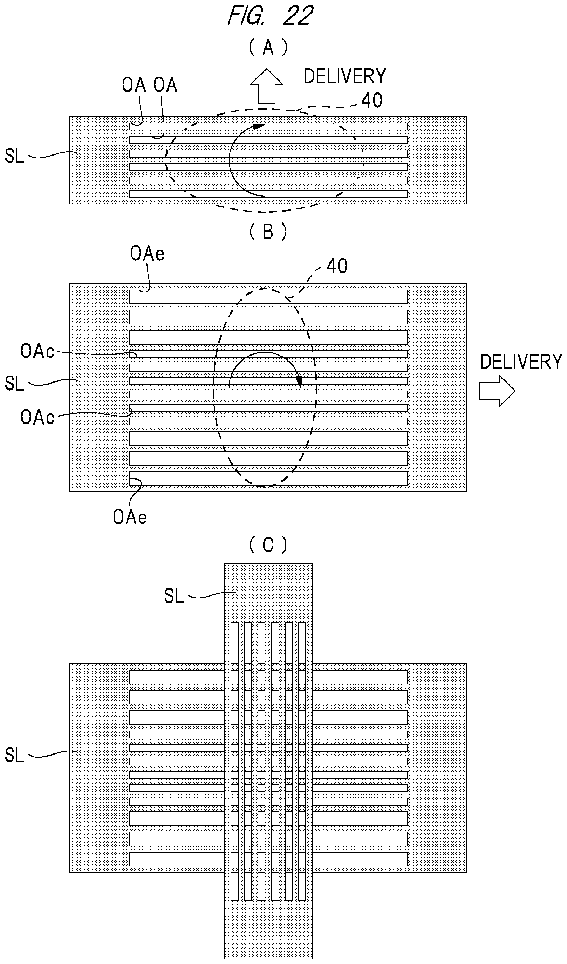

FIG. 22 is a diagram showing one example of a configuration of the slit. For example, as shown in FIG. 22 (A), a slit in which the linear opening having a width of 1.5 mm is arranged at a pitch of 1.5 mm may be used. In this case, the slit SL can be arranged so that the opening OA extends in the minor radius direction of the egg that is the process-target object 40, and the egg can be rotated in the minor radius direction.

For example, as shown in FIG. 22 (B), a slit in which an opening OAc on the center has a width of 0.5 mm while an opening OAe near each of the both ends has a width of 1.0 mm may be used. A width of a gap (shielding portion) between the openings is 0.5 mm. Note that the width of the gap (shielding portion) between the openings may be large and small (have a dense portion and an isolate portion).

In this case, the slit SL can be arranged so that the opening extends in the minor radius direction of the egg that is the process-target object 40, and the egg can be rotated in the minor radius direction.

For example, the width of the gap (shielding portion) between the openings can be adjusted in a range of 0.3 mm to 3 mm, and the width of the opening can be adjusted in a range of 0.3 mm to 3 mm, and it is preferable to set the slit so that the surface dose of each portion on the surface of the process-target object (such as the egg) 40 is within 1 kGy to 3 kGy.

In the second embodiment, the egg that is the process-target object 40 is delivered while rotating. However, the process-target object 40 may be irradiated with the electron beam while rotating at a fixed position.

For example, as shown in FIG. 22 (C), overlap of the slit shown in FIG. 22(A) and the slit shown in FIG. 22(B) may be used. In this case, the gap (shielding portion) between the openings exists in a mesh form. Therefore, a metallic plate having such a mesh form may be used as the slit.

### (Third Application Example)

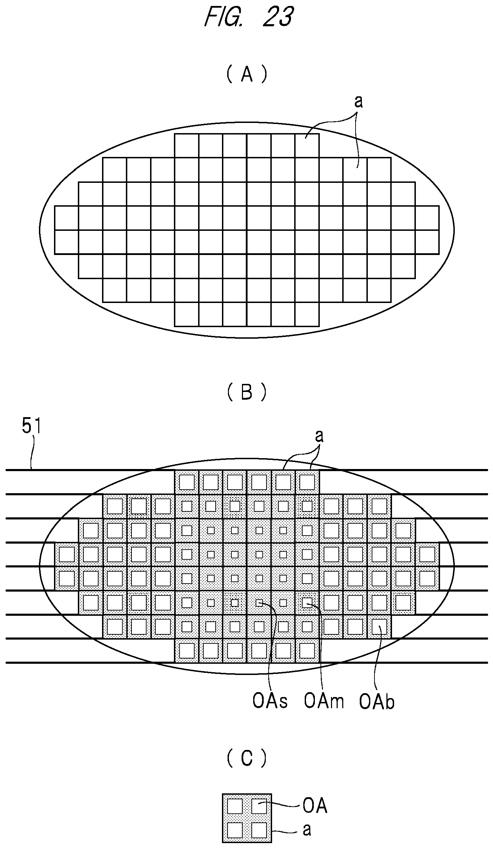

A slit may be designed so that a height of a predetermined section of the process-target object (such as the egg) 40 corresponds to an area of the opening. FIG. 23 is a diagram showing relation between the height of the area of the process-target object and the area of the opening.

As shown in FIG. 23 (A), a plurality of areas "a" are set in a plan view of the process-target object (such as the egg) 40 viewed from above. The opening of the slit is set to correspond to an average height of the process-target objects (such as the egg) 40 inside the areas "a". For example, if the average height is equal to or higher than a first criterion value, an opening rate is defined to be about 30%. And, if the average height is between the first criterion value and a second criterion value, the opening rate is defined to be about 60%. If the average height is equal to or lower than the second criterion value, the opening rate is defined to be about 90%. In other words, if the average height of the process-target objects (such as the egg) 40 inside the areas "a" is large, a small opening OAs is set. If the average height is middle, a middle opening OAm is set. If the average height is small, a big opening OAb is set (see FIG. 23(B)). In FIG. 23(B), note that the number of the openings OA corresponding to the areas "a" is one. However, as shown in FIG. 23(C), the number of the openings OA corresponding to the areas "a" may be plural, and a total area may be defined as the opening rate.

In this manner, the opening of the slit can be set in accordance with the surface shape (three-dimensional shape) of the process-target object 40.

### (Fourth Application Example)

In the working example, the raw egg has been explained as the example of the process-target object 40. However, the process-target object (irradiation-target object) needs to be only a food having the eatable part and the surface part (such as the shell or the outer skin) covering the eatable part, and the sterilizing device and the sterilizing method of the present embodiment can be preferably used for the sterilizing process of such foods.

As such foods, not only the eggs but also fruits, crustaceans and others are exemplified. As the fruits, for example, oranges, lemons, grapefruits, apples, pears, grapes, peaches and others are exemplified. As the crustaceans, shrimps, crabs and others are exemplified. The lemons and the grapefruits have surface asperity (holes) on its outer surface, and the sterilizing method of the present embodiment is preferably used for these foods.

Also, the substantially spherical or oval spherical citrus fruits or cylindrical objects can be irradiated while rotating, and therefore, the sterilizing method of the second embodiment is applicable to these objects.

As application to other items than the foods, the sterilization and the disinfection of medical containers, food packing medium and others made of plastic, paper, glass or others are exemplified. For these items, it is unnecessary to consider the dose of the bremsstrahlung X ray on the irradiation-target parison (object), and therefore, the method is effective for suppressing the partial deterioration of the radiant-ray irradiation or the partial deterioration of the quality of the parison (object) due to unevenness of the irradiation. Particularly, the sterilizing device and the sterilizing method are preferable to the mold-resistant process for the substantially spherical or oval spherical citrus fruits and the cylindrical food packages. In the foregoing, the invention made by the present inventors has been concretely described on the basis of the embodiments. However, it is needless to say that the present invention is not limited to the foregoing embodiments, and various modifications and alterations can be made within the scope of the present invention.

### EXPLANATION OF REFERENCE CHARACTERS

- 1: yolk
- 2: albumen
- 3: chalaza
- 4: eggshell
- 5: cuticular layer
- 6: pore
- 7: eggshell membrane
- 10: electron-beam generator unit
- 12: terminal
- 12a: filament
- 12c: grid
- 16c: accelerating direct-current power supply
- 20: irradiation chamber
- 21: delivery unit
- 21a: roller
- 21b: space (gap)
- 21c: circular truncated cone member
- 21d: shaft
- 22: placement stage
- 24: beam collector
- 28: vacuum exhaust system
- 29: blower
- 30: irradiation window unit
- 32: window foil
- 34: window frame unit
- 40: process-target object
- 50: bar
- 50s: spacer
- a: area
- C: indicator label
- CD: agar
- EB: electron beam
- L: lane
- MF: metallic film
- OA: opening
- PL: supporting member
- P: pore
- Q: concave portion
- SL: slit
- XL: bremsstrahlung X ray

## Claims

1. A sterilizing device configured to sterilize a process-target object by irradiating the process-target object with electron beam, comprising:
an electron-beam irradiator unit configured to irradiate the process-target object with the electron beam;
a placement unit for the process-target object; and
a slit unit arranged between the process-target object and the electron-beam irradiator unit,
wherein the slit unit includes a plurality of openings and is arranged to be upper than a high portion of the process-target object.

2. The sterilizing device according to claim 1,
wherein the process-target object includes the high portion and a low portion lower than the high portion, and
an opening rate in a second region of the slit unit corresponding to the high portion is smaller than an opening rate in a first region of the slit unit corresponding to the low portion.

3. The sterilizing device according to claim 1,
wherein the process-target object is a food having an eatable part and a surface part covering the eatable part, and
the slit unit is made of a metal material or a carbon material including a plurality of linear openings.

4. The sterilizing device according to claim 3,
wherein the process-target object includes the high portion and a low portion lower than the high portion, and
a width of the linear opening of the slit unit on the high portion is smaller than a width of the linear opening of the slit unit on the low portion.

5. The sterilizing device according to claim 1,
wherein the electron-beam irradiator unit includes:
a chamber;
a filament arranged in the chamber; and
a window arranged in the chamber, and
the irradiation with the electron beam is made through the window when electrons that are released by flow of electric current through the filament are accelerated by an acceleration voltage.

6. The sterilizing device according to claim 1,
wherein the placement unit is a delivery unit configured to deliver the process-target object while rotating it so that the process-target object passes in a region irradiated with the electron beam from the electron-beam irradiator unit.

7. The sterilizing device according to claim 6,
wherein the delivery unit includes a plurality of rollers supported about an axis, and
the process-target object is delivered while rotating in an opposite direction to a rotation direction of the roller.

8. The sterilizing device according to claim 1,
wherein a surface dose on a process-target object is equal to or higher than 0.1 kGy and equal to or lower than 10 kGy.

9. The sterilizing device according to claim 6,
wherein an X-ray dose on an eatable part of a process-target object is equal to or lower than 0.1 Gy.

10. The sterilizing device according to claim 1,
wherein the process-target object is a raw egg.

11. A sterilizing method configured to sterilize a process-target object having an eatable part and a surface part covering the eatable part by irradiating the process-target object with electron beam, comprising steps of:
(a) preparing an electron-beam irradiator including an electron-beam irradiator unit configured to irradiate the process-target object with the electron beam, a placement unit for the process-target object, and a slit unit arranged between the process-target object and the electron-beam irradiator unit; and
(b) placing the process-target object on the placement unit and irradiating the process-target object with the electron beam through the slit unit,
wherein the slit unit of the step (b) includes a plurality of openings and is arranged to be upper than a high portion of the process-target object.

12. The sterilizing method according to claim 11,
wherein the process-target object includes the high portion and a low portion lower than the high portion, and,
an opening rate in a second region of the slit unit corresponding to the high portion is smaller than an opening rate in a first region of the slit unit corresponding to the low portion.

13. The sterilizing method according to claim 11,
wherein the process-target object is a food having an eatable part and a surface part covering the eatable part, and
the slit unit is made of a metal material or a carbon material including a plurality of linear openings.

14. The sterilizing method according to claim 13,
wherein the process-target object includes the high portion and a low portion lower than the high portion, and
a width of the linear opening of the slit unit on the high portion is smaller than a width of the linear opening of the slit unit on the low portion.

15. The sterilizing method according to claim 11,
wherein the placement unit is a delivery unit configured to deliver the process-target object while rotating it so that the process-target object passes in a region irradiated with the electron beam from the electron-beam irradiator unit, and
the step (b) is a step of irradiating the process-target object with the electron beam while rotating the process-target object.

16. The sterilizing method according to claim 14,
wherein the process-target object includes the high portion and a low portion lower than the high portion, and
a width of the linear opening of the slit unit on the high portion and a width of the linear opening of the slit unit on the low portion are set under simulation based on a surface shape of the process-target object.

17. The sterilizing method according to claim 11,
wherein a surface dose on a process-target object is equal to or higher than 0.1 kGy and equal to or lower than 10 kGy.

18. The sterilizing method according to claim 11,
wherein an X-ray dose on an inside of a process-target object is equal to or lower than 0.1 Gy.

19. The sterilizing method according to claim 11,
wherein the process-target object is a raw egg.

20. The sterilizing method according to claim 15,
wherein a rotation speed of the process-target object is 0.3 rotation/seconds to 5 rotation/seconds, and
a delivery speed of the process-target object is 0.1 m/minutes to 5 m/minutes.
